# DEMANDE DE BREVET EUROPEEN

(11) **EP 0 534 860 A1**
(43) Date de publication de la demande: **31.03.1993**
(21) Numéro de dépôt: 92402632.1
(22) Date de dépôt: 25.09.1992
(51) Int. Cl.: A61K 31/47, A61K 31/495, A61K 9/08

(54) **Solutions injectables stables de sparfloxacine ou de ses sels**

(30) Priorité: 27.09.1991 FR 9111913
(71) Demandeur: RHONE-DPC EUROPE, 92160 Antony (FR)
(72) Inventeur: Conrath, Guillaume, F-92160 Antony (FR)
(74) Mandataire: Savina, Jacques

(57) **Abrégé**

Composition pharmaceutique constituée d'une solution aqueuse stable de la sparfloxacine de formule : 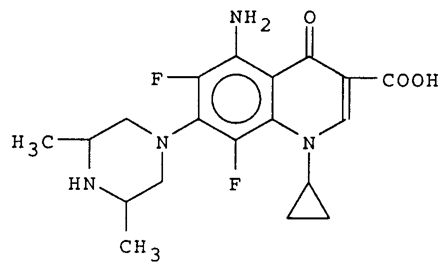
contenant
- la sparfloxacine,
- un ou plusieurs polyhydroxyacides monocarboxyliques ou leurs dérivés lactone, au moins en quantité stoechiométrique par rapport à la sparfloxacine,
- si nécessaire un excès du polyhydroxyacide monocarboxylique ou d'un autre acide pharmaceutiquement acceptable destiné à assurer un pH de solubilisation complète du sel ainsi formé, inférieur ou égal à 5,
- éventuellement un agent isotonisant et/ou d'autres adjuvants pharmaceutiquement acceptables.

## Description

La présente invention concerne une nouvelle composition pharmaceutique convenable pour l'administration parentérale de la sparfloxacine.

Dans la demande de brevet EP 221 463 a été décrite la sparfloxacine de formule : 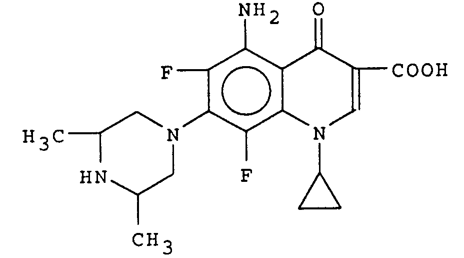
ainsi que ses sels d'addition avec des acides ou des bases, utile comme agent antimicrobien.

La demande de brevet EP 284935 décrit aussi des quinolones et leurs sels et parmi ces produits, la sparfloxacine.

Malheureusement les sels de la sparfloxacine sont généralement insolubles ou instables en solution. Il n'avait donc pas été possible jusqu'à présent de préparer des compositions convenables pour l'administration parentérale.

Le brevet européen EP 322 892 mentionne ces difficultés et décrit des compositions lyophilisées contenant un sel de la sparfloxacine avec un acide. Néanmoins ces compositions ne permettent pas d'obtenir des solutions stables pendant un temps prolongé et de ce fait seule la mise en solution extemporanée est possible. De plus de telles solutions risquent d'être impropres à un usage en perfusions lentes.

La présente invention concerne des solutions injectables stables. Ces compositions peuvent être conservées pendant un temps prolongé et sont en général stables à la chaleur.

Les solutions selon l'invention sont des solutions aqueuses contenant
- la sparfloxacine,
- un ou plusieurs polyhydroxyacides monocarboxyliques ou leurs dérivés lactone, au moins en quantité stoechiométrique par rapport à la sparfloxacine,
- si nécessaire un excès du polyhydroxyacide monocarboxylique ou d'un autre acide pharmaceutiquement acceptable destiné à assurer un pH de solubilisation complète du sel ainsi formé, inférieur ou égal à 5,
- éventuellement un agent isotonisant et/ou d'autres adjuvants pharmaceutiquement acceptables.

Les solutions selon l'invention contiennent au moins 1% de sparfloxacine. Elles peuvent néanmoins contenir des concentrations pouvant aller jusqu'à 40% de sparfloxacine. Il est bien entendu que des solutions de concentration inférieure à 1% sont également réalisables et cliniquement utilisables ; ces solutions entrent également dans le cadre de la présente invention.

Le polyhydroxyacide monocarboxylique est choisi de telle manière que son pK_{A} soit supérieur à 3, à 25°C. A titre d'exemple il peut être choisi parmi les acides lactobionique, glucoheptonique, gluconique ou ascorbique

La quantité du polyhydroxyacide monocarboxylique est fonction de la quantité de sparfloxacine. Elle est déterminée de manière à avoir au moins les proportions stoechiométriques et de telle manière à obtenir de préférence des solutions dont le pH n'est pas inférieur à 3,5.

L'acide capable d'assurer une solubilisation complète peut être choisi parmi les acides pharmaceutiquement acceptables qui ne risquent pas, de par leur nature et/ou leur concentration, de déplacer de façon importante le sel de la sparfloxacine avec le polyhydroxyacide monocarboxylique. A titre d'exemple peuvent être choisis des acides organiques comme l'acide acétique, l'acide propionique, l'acide tartrique, l'acide succinique ou les hydroxy ou polyhydroxyacides monocarboxyliques. Des acides plus forts peuvent également être utilisés comme notamment les acides sulfoniques (acide méthanesulfonique par exemple), les acides carboxyliques (acide maléique, acide oxalique, acide malonique par exemple), ou les acides minéraux (par exemple acide chlorhydrique, acide phosphorique, acide sulfurique) dans les cas où leur concentration pourra être fixée de telle manière qu'ils ne risquent pas de déplacer de façon importante le sel de la sparfloxacine avec le polyhydroxyacide monocarboxylique. Il est néanmoins entendu que pour des questions de commodité, il est tout à fait avantageux d'ajuster la solubilité et le pH des solutions au moyen du polyhydroxyacide monocarboxylique employé.

Le pH des solutions est inférieur ou égal à 5. D'une manière générale le pH des solutions doit être compatible avec une administration directe. De préférence il est compris entre 3,5 et 5.

De préférence les solutions selon l'invention sont isotoniques. Néanmoins les solutions non isotoniques destinées à une injection dans une poche de perfusion au glucose entrent également dans le cadre de la présente invention.

Les solutions selon l'invention peuvent être isotonisées par addition d'un agent isotonisant tel que par exemple le glucose, le glycérol, le sorbitol, le mannitol, le xylitol, le fructose, ou le lactose.

Les solutions selon l'invention sont plus spécialement destinées à l'administration par voie parentérale. Elles peuvent être également utilisées par voie orale, occulaire, auriculaire ou en application locale sur la peau et les muqueuses.

Il est entendu que les solutions selon l'invention peuvent contenir, outre l'isotonisant, d'autres adjuvants compatibles et pharmaceutiquement acceptables. A titre d'exemple elles peuvent également contenir des édulcorants, des arômes, des agents de conservation des colorants ou éventuellement des gélifiants.

Il est entendu que les sels de la sparfloxacine avec le polyhydroxyacide monocarboxylique ainsi obtenus, qui permettent la préparation et l'usage de solutions injectables, stables, entrent aussi dans le cadre de la présente invention.

Les solutions selon l'invention sont préparées alternativement par addition d'eau à un mélange comprenant la sparfloxacine, le/les polyhydroxyacides monocarboxyliques choisis, en quantité au moins stoechiométrique par rapport à la sparfloxacine, le cas échéant l'excès du polyhydroxyacide monocarboxylique ou l'autre acide destiné à assurer un pH de solubilisation du sel de la sparfloxacine inférieur ou égal à 5 et/ou l'isotonisant et les autres adjuvants, ou bien par addition de sparfloxacine et éventuellement des autres additifs à une solution du/des polyhydroxyacides monocarboxyliques.

La préparation et la répartition de la solution sont effectuées de préférence sous azote. Les solutions ainsi obtenues peuvent être stérilisées à la chaleur (stérilisation à l'autoclave). Dans le cas des solutions de sel de l'acide ascorbique, il est néanmoins préférable d'opérer par filtration stérilisante.

Il est également possible de préparer les solutions selon l'invention au moyen de dérivés lactone qui par hydrolyse génèrent in situ les polyhydroxyacides monocarboxyliques.

Les solutions selon l'invention présentent l'avantage d'une très bonne stabilité physico-chimique.

Elles sont particulièrement intéressantes dans la mesure où elles donnent accès à des formulations liquides de la sparfloxacine ce qui jusqu'à présent n'avait pas été réalisable, notamment à une formulation injectable, stable à la lumière dans des conditions normales d'utilisation et stable à la chaleur tout en ayant un pH compatible avec une administration directe. Cette nouvelle formulation est particulièrement avantageuse pour le stockage, la commodité et la rapidité d'utilisation d'une forme prête à l'emploi et aussi dans le cas des perfusions lentes. Par ailleurs, et compte tenu de la bonne solubilité des sels obtenus dans les conditions de pH utilisées, ces solutions peuvent le cas échéant être concentrées, ce qui permet un choix varié des doses à formuler.

Les exemples suivants donnés à titre non limitatif illustrent la présente invention.

### Exemple 1

### Préparation d'une solution à 4 mg/ml de sparfloxacine, isotonisée au glucose.

400 mg de sparfloxacine sont mélangés à 321 mg de glucono δ-lactone et 4,75 g de glucose monohydraté. Ce mélange de poudres est dissous dans de l'eau pour préparation injectable préalablement inertée à l'azote. La dissolution est obtenue par agitation sous azote. Le volume est complété à 100 ml avec de l'eau pour préparation injectable. La solution ainsi obtenue est stérilisée à l'autoclave (120°C, 20 minutes).

Le pH de la solution est de 3,8.

Cette solution reste limpide après 5 mois de conservation à 4, 20 et 35°C, sans modification de pH.

### Exemple 2

### Préparation d'une solution à 10 mg/ml de sparfloxacine isotonisée au glucose.

En opérant comme précédemment à l'exemple 1, mais en ajustant le volume à 40 ml et en employant 1,74 g de glucose monohydraté, on obtient une solution limpide dont le pH est de 3,8.

Une telle solution préparée à l'échelle de 2,5 litres reste limpide est n'est pas modifiée en pH sur une période d'au moins 5 mois et dans les conditions de température suivantes : 20°C, 4°C, et 35°C.

Une analyse HPLC effectuée après 3 mois de conservation à 20°C, 4°C 35°C et 45°C n'a pas montré de dégradation du produit.

### Exemple 3

### Préparation d'une solution à 20 mg/ml de sparfloxacine isotonisée au glucose.

En opérant comme précédemment à l'exemple 1 mais en ajustant le volume à 20 ml et par addition de 770 mg de glucose monohydraté, on prépare une solution limpide dont le pH est 3,7.

Cette solution préparée à l'échelle de 2 litres reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 4

### Préparation d'une solution à 50 mg/ml de sparfloxacine isotonisée au glucose.

En opérant comme à l'exemple 1, mais en ajustant le volume à 8 ml et par addition de 130 mg de glucose monohydraté, on prépare une solution limpide dont le pH est 3,5.

Cette solution préparée à l'échelle de 2 litres reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 5

### Préparation d'une solution à 100 mg/ml de sparfloxacine, hypertonique.

En opérant comme à l'exemple 1, mais en ajustant le volume à 4 ml et sans addition de glucose, on prépare une solution dont le pH est de 3,5.

Cette solution préparée à l'échelle de 1 litre reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 6

### Préparation d'une solution à 200 mg/ml de sparfloxacine, hypertonique.

En opérant comme à l'exemple 1, mais en ajustant le volume à 2 ml et sans ajouter de glucose, on prépare une solution limpide dont le ph est 3,5.

Cette solution préparée à l'échelle de 1 litre reste limpide et n'est pas modifiée en pH après 3 mois de conservation à 20, 4, 35 et 45°C.

L'analyse HPLC effectuée après 1 mois de conservation à 20, 35 et 45°C ne révèle pas de produit de dégradation.

### Exemple 7

### Préparation d'une solution à 10 mg/ml de sparfloxacine.

1000 mg de sparfloxacine sont mélangés à 1,79 g d'acide lactobionique. Ce mélange de poudres est dissous dans de l'eau pour préparation injectable préalablement inertée à l'azote. La dissolution est obtenue par agitation sous azote. Le volume est complété à 100 ml avec de l'eau pour préparation injectable. La solution ainsi obtenue est stérilisée à l'autoclave (120°C, 20 minutes).

Le pH de la solution est de 3,75.

Cette solution conservée 5 mois à 4 et à 20°C reste limpide et sans modification de pH.

Un dosage par HPLC après 5 mois de conservation ne montre pas d'impureté de dégradation.

### Exemple 8

### Préparation d'une solution à 20 mg/ml de sparfloxacine.

En opérant comme à l'exemple 7, mais à partir de 2000 mg de sparfloxacine et de 3,58 g d'acide lactobionique et en ajustant le volume à 100 ml, on obtient une solution limpide de pH = 3,5.

### Exemple 9

### Préparation d'une solution à 30 mg/ml de sparfloxacine.

En opérant comme à l'exemple 7, mais à partir de 3000 mg de sparfloxacine et de 8, 96 g d'acide lactobionique et en ajustant le volume à 100 ml, on obtient une solution limpide de pH = 3,5.

### Exemple 10

### Préparation d'une solution à 10 mg/ml de sparfloxacine.

En opérant comme à l'exemple 7, mais à partir de 1000 mg de sparfloxacine et de 2,26 g d'acide glucoheptonique et en ajustant le volume à 100 ml, après chauffage à 50°C pendant 1 heure, on obtient une solution limpide de pH = 4,3.

### Exemple 11

### Préparation d'une solution à 10 mg/ml de sparfloxacine isotonisée au glucose.

1000 mg de sparfloxacine sont mélangés à 528 mg d'acide ascorbique et 4 g de glucose anhydre. Ce mélange de poudres est dissous dans de l'eau pour préparation injectable préalablement inertée à l'azote. La dissolution est obtenue par agitation sous azote. Le volume est complété à 100 ml avec de l'eau pour préparation injectable. La solution ainsi obtenue est stérilisée par filtration stérilisante.

Le pH de la solution est de 4,8.

Cette solution conservée 7 mois à 4 et à 20°C, à l'abri de la lumière, reste limpide et sans modification de pH.

Un dosage par HPLC après 7 mois de conservation ne montre pas d'impureté de dégradation.

### Exemple 12

### Préparation d'une solution à 10 mg/ml de sparfloxacine, isotonisée au glycérol.

En opérant comme à l'exemple 11, mais à partir de 2,2 g de glycérol au lieu du glucose, on obtient une solution limpide de pH = 5.

Cette solution reste limpide après 7 mois à 4 et 20°C, à l'abri de la lumière et sans modification de pH.

Un dosage par HPLC après 7 mois de conservation ne montre pas d'impureté de dégradation.

### Exemple 13

### Préparation d'une solution à 20 mg/ml de sparfloxacine.

En opérant comme à l'exemple 11, mais à partir de 2000 mg de sparfloxacine et de 1,76 g d'acide ascorbique, on obtient une solution limpide de pH = 4,2.

Cette solution reste limpide après 7 mois à 20°C, à l'abri de la lumière.

## Revendications

**1-** Solution aqueuse stable de la sparfloxacine de formule : 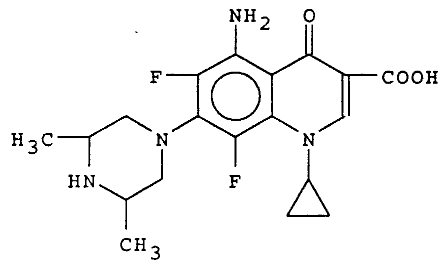 caractérisée en ce qu'elle contient :
- la sparfloxacine,
- un ou plusieurs polyhydroxyacides monocarboxyliques ou leurs dérivés lactone, au moins en quantité stoechiométrique par rapport à la sparfloxacine,
- si nécessaire un excès du polyhydroxyacide monocarboxylique ou d'un autre acide pharmaceutiquement acceptable destiné à assurer un pH de solubilisation complète du sel ainsi formé, inférieur ou égal à 5,
- éventuellement un agent isotonisant et/ou d'autres adjuvants pharmaceutiquement acceptables.

**2-** Une solution selon la revendication 1, caractérisée en ce que le polyhydroxyacide monocarboxylique est choisi parmi les acides ayant un pK_{A} supérieur à 3 à 25°C.

**3-** Une solution selon la revendication 1 ou 2, caractérisée en ce que le polyhydroxyacide monocarboxylique est choisi parmi :
- l'acide lactobionique,
- l'acide glucoheptonique,
- l'acide gluconique ou
- l'acide ascorbique.

**4-** Une solution selon la revendication 1, caractérisée en ce qu'elle contient une concentration allant jusqu'à 40% de sparfloxacine.

**5-** Une solution selon la revendication 1, caractérisée en ce que son pH est compris entre 3,5 et 5.

**6-** Une solution selon la revendication 1, caractérisée en ce que l'isotonisant est choisi parmi le glucose, le glycérol, le sorbitol, le mannitol, le xylitol, le fructose, ou le lactose.

**7-** Préparation d'une solution selon la revendication 1, caractérisée en ce qu'un mélange de sparfloxacine, d'un ou plusieurs polyhydroxyacides monocarboxyliques en quantité au moins stoechiométrique par rapport à la sparfloxacine et éventuellement d'un excès du polyhydroxyacide monocarboxylique ou d'un autre acide capable d'assurer un pH de solubilisation complète du sel inférieur ou égal à 5 et/ou d'un isotonisant et d'autres adjuvants est additionné d'eau.

**8-** Préparation d'une solution selon la revendication 1, caractérisée en ce que la sparfloxacine, éventuellement un acide capable d'assurer un pH de solubilisation complète du sel inférieur ou égal à 5 et/ou un isotonisant et d'autres adjuvants sont ajoutés à une solution d'un ou plusieurs polyhydroxyacides monocarboxyliques.

**9-** Un sel de la sparfloxacine avec un polyhydroxyacide monocarboxylique choisi parmi :
- l'acide lactobionique,
- l'acide glucoheptonique,
- l'acide ascorbique.

**10-** Composition pharmaceutique caractérisée en ce qu'elle contient une solution selon la revendication 1, éventuellement en association avec un ou plusieurs diluants ou adjuvants compatibles et pharmaceutiquement acceptables.
